# FASCICULE DE BREVET EUROPEEN

(11) **EP 3 498 262 B1**
(45) Date de publication et mention de la délivrance du brevet: **01.09.2021**
(21) Numéro de dépôt: 19156054.9
(22) Date de dépôt: 02.07.2008
(51) Int. Cl.: A61K 9/06, A61P 19/00, A61K 31/728, A61K 47/10

(54) **GEL INJECTABLE D'ACIDE HYALURONIQUE**
INJIZIERBARES HYALURONSÄUREGEL
HYALURONIC ACID INJECTABLE GEL

(30) Priorité: 02.07.2007 FR 0704772
(43) Date de publication de la demande: 19.06.2019
(62) Demande divisionnaire de: 08827889.0
(73) Titulaire: Aptissen SA, 1228 Plan-Les-Ouates (CH)
(72) Inventeur: GAVARD MOLLIARD, Samuel, 74800 LA ROCHE SUR FORON (FR); BENOIT, Olivier, 74330 EPAGNY (FR)
(74) Mandataire: Ipsilon

(56) Documents cités:
- EP-A- 0 297 860
- EP-A- 0 499 164
- WO-A-2006/067608
- WO-A1-2005/097226
- FR-A- 2 865 737
- GB-A- 2 196 255
- US-A- 5 095 037
- US-A- 5 972 909
- US-A1- 2003 203 030
- CROMAPHARMA: "MEGACROM", 20110101, août 2002 (2002-08), page 1, XP003028282,
- MAZZUCCO D ET AL: "Visiol", TRB CHEMEDICA OPHTHALMIC LINE,, 1 janvier 2011 (2011-01-01), pages 1-2, XP003028283,
- BELDA J I ET AL: "Hyaluronic acid combined with mannitol to improve protection against free-radical endothelial damage: Experimental Model", JOURNAL CATARACT AND REFRACTIVE SURGERY, SURGERY, FAIRFAX, VA, US, vol. 31, no. 6, 1 juin 2005 (2005-06-01), pages 1213-1218, XP027831600, ISSN: 0886-3350 [extrait le 2005-06-01]
- MAZZUCCO D ET AL: "Rheology of joint fluid in total knee arthroplasty patients", JOURNAL OF ORTHOPAEDIC RESEARCH, ORTHOPAEDIC RESEARCH SOCIETY, US, vol. 20, 1 janvier 2002 (2002-01-01), pages 1157-1163, XP003028284, ISSN: 0736-0266
- Unknown: "Extract of a product information: Lubravisc- Sodium Hyaluronate", , 1 January 2006 (2006-01-01), pages 1-1, XP055813696, Retrieved from the Internet: URL:www.bohusbiotech.com
- MENDOZA ET AL: "Inhibitory effects of different antioxidants on hyaluronan depolymerization", CARBOHYDRATE RESEARCH, PERGAMON, GB, vol. 342, no. 1, 19 December 2006 (2006-12-19), pages 96-102, XP005808572, ISSN: 0008-6215, DOI: 10.1016/J.CARRES.2006.10.027

## Description

L'invention concerne une formulation aqueuse injectable stérile sous forme de gel composée d'acide hyaluronique (ou l'un de ses sels) avec ou sans autre(s) polysaccharide(s) d'origine naturelle et d'un ou plusieurs polyol(s). Cette formulation injectable est utilisée en intra-articulaire dans le traitement des dégénérescences articulaires. L'invention concerne également le procédé de préparation de ladite formulation.

Une articulation est une jonction permettant de relier deux os et de leur donner une mobilité l'un par rapport à l'autre.

Les articulations synoviales sont les articulations les plus nombreuses, notamment au niveau des membres. Dans ces articulations, les os s'unissent via une cavité remplie d'un liquide à la fois visqueux et élastique appelé liquide synovial.

Le liquide synovial est responsable du bon fonctionnement et de la protection des articulations. Il est notamment constitué d'un polysaccharide, l'acide hyaluronique, qui confère au liquide synovial des propriétés de viscoélasticité permettant, en fonction des contraintes imposées, une lubrification de l'articulation ou une absorption des chocs.

Dans le cas des dégénérescences articulaires comme l'arthrose du genou (dégénérescences dues notamment à des facteurs comme l'obésité, l'hérédité, les traumatismes, ...), le liquide synovial se dégrade (diminution de la concentration et de la masse moléculaire de l'acide hyaluronique) et cette dégradation réduit la capacité du liquide synovial à lubrifier l'articulation et à amortir les chocs.

Le traitement par viscosupplémentation consiste à injecter un gel dans l'articulation afin de remplacer le liquide synovial déficient. La viscosupplémentation peut atténuer ou enrayer la douleur et contribuer à restaurer la mobilité de l'articulation.

Les produits de viscosupplémentation actuellement sur le marché sont des gels qui contiennent de l'acide hyaluronique. Ces gels peuvent être à base d'acide hyaluronique d'origine animale ou non animale et réticulés (cas de Synvisc®, Durolane®) ou non réticulés (cas de Synocrom®, Arthrum®, Lubravisc®, Structovial®).

Il est bien connu de l'homme de l'art que la rémanence d'un gel à base d'acide hyaluronique est faible dans une articulation (de quelques heures à quelques jours). D'après Laurent «The Chemistry, biology and medical applications of hyaluronan and its derivatives, Wenner-Gren International series, Volume 72 », la demi-vie dans une articulation de lapin d'une solution à 1 % de hyaluronan est de 12h et celle d'un gel à 0.5% de Hylan B est de 9 jours.

Cette faible rémanence (cinétique de résorption rapide du gel au sein de l'articulation) s'explique par une dégradation (par dépolymérisation) de l'acide hyaluronique. Les principaux facteurs de dégradation de l'acide hyaluronique dans l'articulation sont la dégradation radicalaire, la dégradation thermique à 37°C et la dégradation mécanique (la dégradation enzymatique n'est pas un facteur important de dégradation dans l'articulation). Bien que l'efficacité thérapeutique du viscosupplément soit de plus longue durée que son temps de résidence dans l'articulation, la rémanence d'un gel à base d'acide hyaluronique dans l'articulation est un paramètre prépondérant régissant l'efficacité du produit. Ainsi, plus le gel à base d'acide hyaluronique à un temps de résidence important dans l'articulation et plus le traitement par viscosupplémentation (diminution de la douleur, gain de mobilité) est efficace. Par conséquent, l'augmentation du temps de résidence (rémanence) d'un gel au sein de l'articulation est un point capital pour augmenter l'efficacité d'un traitement par viscosupplémentation à l'aide d'un gel à base d'acide hyaluronique.

Il est bien connu par l'homme de l'art que l'augmentation de la concentration en acide hyaluronique, l'utilisation de hautes masses moléculaires d'acide hyaluronique et les techniques de réticulation/greffage de l'acide hyaluronique permettent d'améliorer la rémanence d'un gel à base d'acide hyaluronique. Toutefois, l'optimisation des différents paramètres listés ci-dessus ne semble pas suffisante pour permettre d'augmenter significativement la rémanence d'un gel à base d'acide hyaluronique en intra-articulaire (la demi-vie, au sein de l'articulation, des gels actuels de viscosupplémentation présents sur le marché n'est au maximum que de quelques jours).

De façon tout à fait inattendue et surprenante, on a mis en évidence :
- que la présence d'un polyol dans une formulation aqueuse stérile à base d'acide hyaluronique permet d'augmenter significativement la résistance aux dégradations de ce gel
- une forte affinité de l'acide hyaluronique et du polyol au sein du gel stérile impliquant une cinétique lente de relarguage du polyol hors du gel : cette affinité entre l'acide hyaluronique et le polyol implique une protection efficace sur le long terme du gel par le polyol par un effet synergique
- pour une composition particulière d'une formulation aqueuse d'acide hyaluronique et d'un polyol, la stérilisation donne à ce gel des propriétés viscoélastiques tout à fait étonnantes en ce qu'elles reproduisent pratiquement les propriétés viscoélastiques du liquide synovial sain - ces propriétés rhéologiques particulières du gel sont maintenues plus longtemps au cours du temps grâce à la protection contre les dégradations induite par la synergie « acide hyaluronique/polyol ».

La présente invention consiste donc en une formulation aqueuse injectable stérile sous forme de gel composée d'acide hyaluronique (ou l'un de ses sels) avec ou sans autre(s) polysaccharide(s) d'origine naturelle et d'un ou plusieurs polyol(s). Cette formulation, utilisée dans le traitement des dégénérescences articulaires, présente dans certains cas (voir exemples 1 et 3) une rhéologie proche de celle du liquide synovial et toujours une résistance accrue à la dégradation.

L'exemple 4 montre la meilleure résistance d'un gel à base d'acide hyaluronique et d'un polyol lorsque celui-ci est soumis à un test de dégradation radicalaire, thermique et mécanique. Cette meilleure résistance du gel à la dégradation permet une plus longue rémanence du gel injecté en intra-articulaire.

L'exemple 5 montre la meilleure résistance d'un gel à base d'acide hyaluronique et d'un polyol à la dégradation thermique. Cette meilleure résistance du gel à la dégradation thermique permet une plus longue rémanence du gel injecté en intra-articulaire et une meilleure stabilité de la formulation lors du stockage du produit avant utilisation (point important pour la durée de péremption du produit).

L'exemple 8 démontre la forte affinité entre l'acide hyaluronique et un polyol. Injecté dans l'articulation, la forte affinité entre l'acide hyaluronique et le polyol permet une meilleure résistance du gel à la dégradation sur le long terme par un effet synergique. En effet, dans le cas d'une injection d'une solution de polyol dans l'articulation, le lavage naturel va rapidement éliminer la molécule (= polyol) de l'articulation. Dans le cas d'un gel à base d'acide hyaluronique avec polyol, la forte affinité entre l'acide hyaluronique et le polyol va empêcher le relarguage rapide du polyol hors du gel (et donc son élimination rapide hors de l'articulation) et permettre ainsi une protection efficace sur le long terme du gel par le polyol contre les dégradations.

Les exemples 1 et 3 montrent une rhéologie d'un gel à base d'acide hyaluronique et d'un polyol proche de celle du liquide synovial.

La publication MAZZUCCO D. et aL, « Rheology of joint fluid in total knee arthroplasty patients» ; Journal of Orthopaedic Research, 1157-1163, 2002, indique que la fréquence de croisement entre le module élastique G' et le module visqueux G" est égal à 0,41 ± 0,12 Hz pour un liquide synovial sain (non arthosé) du genou. La valeur de cette fréquence de croisement est confirmée par la publication de Fam et aL, « Rheological properties of synovial fluids », Biorheology, 44, 59-74, 2007. Dans cette publication, une figure présente la fréquence de croisement entre les modules G' et G" comprise entre 0 et 10 Hz pour un liquide synovial appartenant à une personne jeune ou âgée ou encore pour un liquide synovial arthrosé.
- En dessous de 0,41 Hz : G" > G', le liquide synovial est à dominante visqueuse impliquant une forte lubrification de l'articulation lorsque le patient est au repos.
- En dessus de 0,41 Hz : G' > G", le liquide synovial est à dominante élastique impliquant une forte absorption des chocs lorsque le patient court ou saute.

La présente invention est exposée dans le jeu de revendications.

Selon la présente invention, la formulation telle que définie dans les revendications possède, après stérilisation à l'autoclave, une fréquence f_{c} de croisement entre le module élastique G' et le module visqueux G" proche de 0,41 Hz. Ainsi, le gel possède des propriétés viscoélastiques proches de celle du liquide synovial.

De ce fait, selon la présente invention :
- Au-dessous de f_{c} : G" > G', le gel est à dominante visqueuse avec une lubrification efficace de l'articulation au repos,
- Au-dessus de f_{c} : G' > G", le gel est à dominante élastique avec une absorption efficace des chocs lorsque le patient court ou saute (protection de l'articulation).

Selon la présente invention, la fréquence de croisement est comprise entre 0 et 10 Hz, de préférence 0,41 ± 0,41 Hz, G' étant supérieur à G" à haute fréquence. Une telle rhéologie est donc appropriée aux contraintes mécaniques des articulations et notamment du genou, de la hanche ou des petites articulations. Par conséquent, elle présente un grand intérêt dans le traitement de l'arthrose par viscosupplémentation du genou ou des autres articulations.

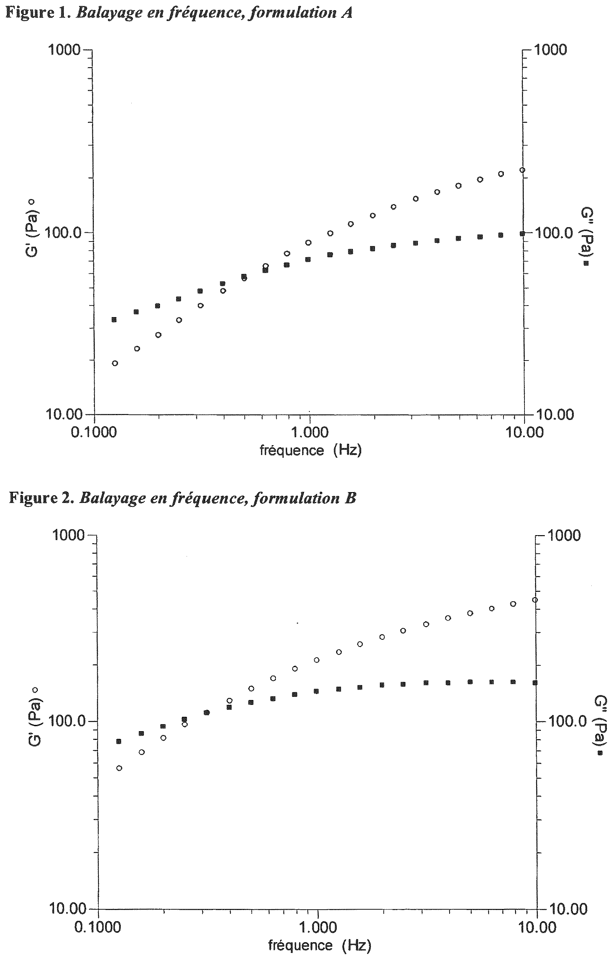

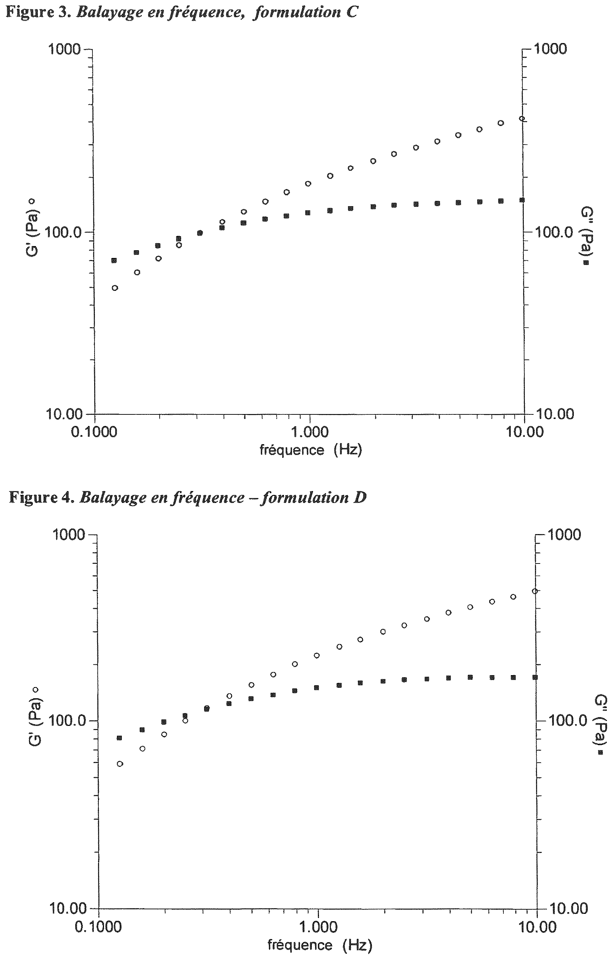

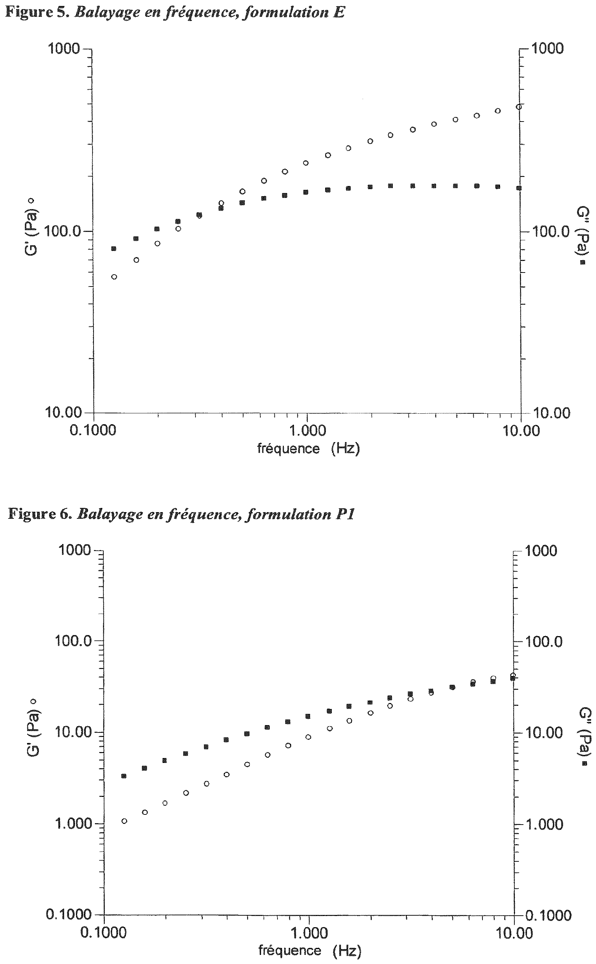

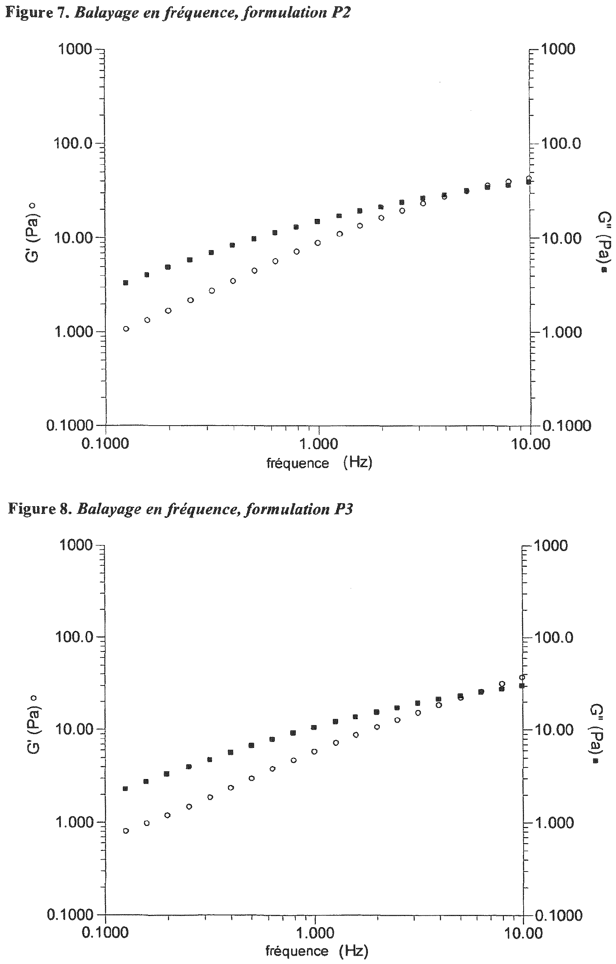

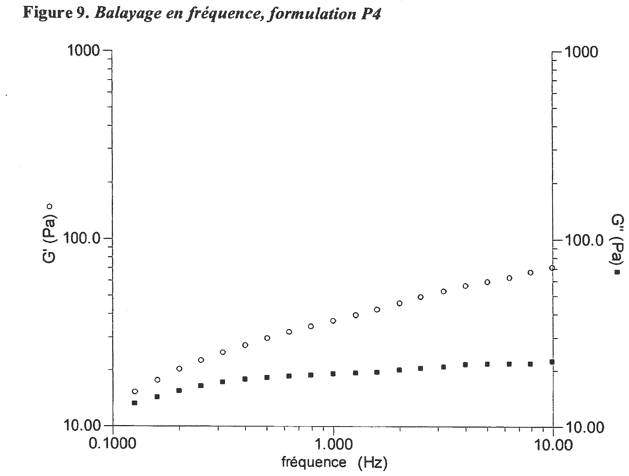

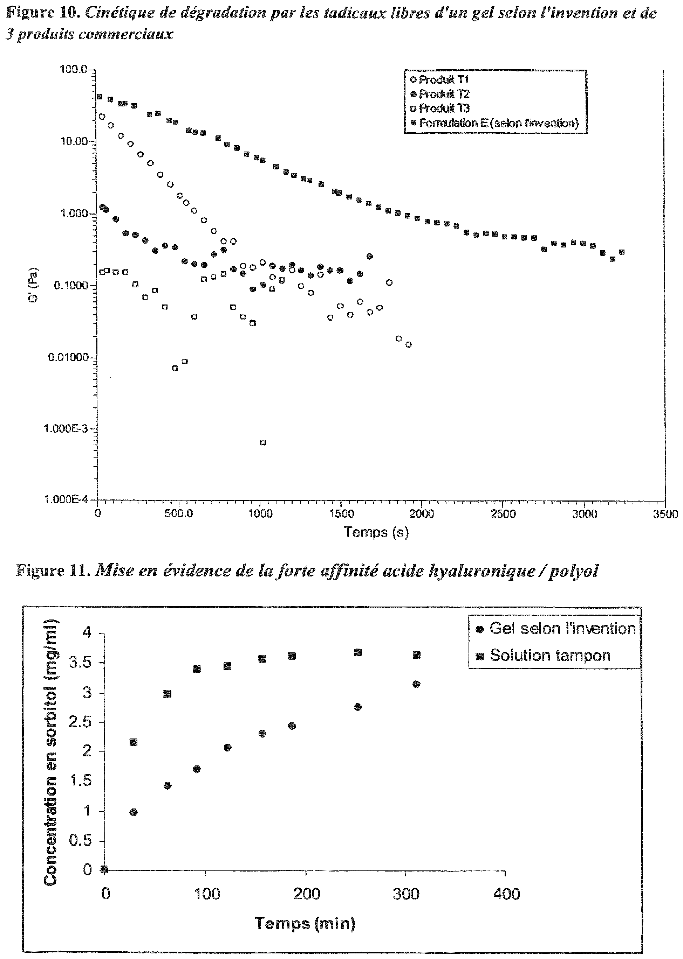

Les figures 1-5 montrent les modules visqueux et élastique de gels selon un aspect de la présente invention tandis que les figures 6-9 montrent ceux de gels du commerce. Les figures 10 et 11 illustrent la cinétique de dégradation radicalaire, thermique et mécanique de gels et l'affinité acide hyaluronique/polyol.

L'acide hyaluronique est préférentiellement obtenu par biofermentation mais il peut également être d'origine animale. Sa masse moléculaire est de préférence de 0,1 à 10x10⁶Da et de préférence encore de 2 à 3x10⁶Da.

La concentration en acide hyaluronique est comprise entre 1 et 100 mg/ml et préférentiellement entre 10 et 25 mg/ml. La concentration en polyols est de préférence de 15 à 45 mg/ml.

Le (ou les) polysaccharide d'origine naturelle pouvant être utilisé en association avec l'acide hyaluronique est choisi par exemple parmi la chondroïtine sulfate, le kératane, le kératane sulfate, l'héparine, l'héparane sulfate, la cellulose et ses dérivés, le chitosan, les xanthanes, les alginates, et l'ensemble de leurs sels respectifs.

L'acide hyaluronique, tout comme le ou les polysaccharide(s) d'origine naturelle, peut être réticulé ou non réticulé, greffé ou non greffé selon les techniques de réticulation/greffage décrites dans l'art antérieur.

Le (ou les) polyol est choisi parmi le propylène glycol, le sorbitol, ou le mannitol.

La concentration en polyol est comprise entre 15 et 100 mg/ml et préférentiellement entre 15 et 45 mg/ml.

La solution aqueuse utilisée est préférentiellement une solution tamponnée. La composition de cette solution tampon est choisie afin d'avoir les propriétés physico-chimiques (pH, osmolarité) et rhéologiques désirées.

Préférentiellement, la solution tampon choisie est une solution tampon phosphate.

Selon la présente invention, la formulation est stérilisée à l'autoclave.

La formulation selon la présente invention est utilisée par injection dans l'articulation et la dose injectée peut être comprise entre 0,1 et 20 ml en fonction de la nature de l'articulation traitée.

A titre illustratif, on donne ci-dessous une formulation de gel viscoélastique que l'on peut préparer selon la présente invention :
- Gel viscoélastique à base d'acide hyaluronique et de sorbitol Solution stérile composée de 20 mg/ml d'acide hyaluronique (MM = 2,5x10⁶ Da) et de 40 mg/ml de sorbitol dans du tampon phosphate.

### Exemples :

Des exemples sont proposés afin d'illustrer l'invention mais ne sont nullement limitatifs de ladite invention. Les formulations préparées dans les exemples suivants sont des gels à base de hyaluronate de sodium (NaHA) non réticulé ou réticulé avec polyol.

La préparation des gels non réticulé ou réticulé est effectuée selon les techniques bien connues par l'homme de l'art. Le hyaluronate de sodium utilisé pour fabriquer ces gels possède une masse moléculaire égale à 2.5x10⁶ Da. Dans le cas des gels réticulés, le réticulant utilisé est le BDDE et la définition du taux de réticulation utilisée est : masse(BDDE)/masse(NaHA sec). L'incorporation du polyol dans le gel est effectuée en ajoutant la quantité nécessaire de polyol dans le gel non réticulé ou réticulé et en mélangeant à la spatule pendant 10 minutes (pour 100 g de gel final).

Les gels préparés sont remplis dans des seringues en verre puis stérilisés à la chaleur humide (T = 121°C).

Le rhéomètre utilisé pour effectuer les mesures rhéologiques est un AR1000 (TA instruments) avec une géométrie plate de 40 mm, un gap de 1000 microns et une température d'analyse de 37°C.

Le dosage des polyols est effectué par une HPLC Ultimate 3000 (Dionex) et une colonne échangeuse d'ions.

### Exemple 1 : Préparation de formulations injectables stériles

Les formulations A et B ne relèvent pas de l'invention revendiquée et ne sont présentes qu'à titre illustratif.
Formulation A : gel à base de NaHA non réticulé avec glycérol
   - 15 mg de NaHA à 2,5x10⁶ Da
   - 20 mg de glycérol
   - Qsp 1 ml de tampon phosphate
Formulation B : gel à base de NaHA non réticulé avec glycérol
   - 20 mg de NaHA à 2,5 x10⁶ Da
   - 20 mg de glycérol
   - Qsp 1 ml de tampon phosphate
Formulation C : gel à base de NaHA non réticulé avec propylène glycol
   - 20 mg de NaHA à 2,5x 10⁶ Da
   - 15 mg de propylène glycol
   - Qsp 1 ml de tampon phosphate
Formulation D : gel à base de NaHA non réticulé avec mannitol
   - 20 mg de NaHA à 2,5 x 10⁶ Da
   - 15 mg de mannitol
   - Qsp 1 ml de tampon phosphate
Formulation E : gel à base de NaHA non réticulé avec sorbitol
   - 20 mg de NaHA à 2,5 X 10⁶ Da
   - 40 mg de sorbitol
   - Qsp 1 ml de tampon phosphate
Formulation F : gel à base de NaHA réticulé avec sorbitol
   - 18 mg de NaHA à 2,5 x 10⁶ Da, taux de réticulation = 6%
   - 50 mg de sorbitol
   - Qsp 1 ml de tampon phosphate

### Exemple 2 : Propriétés physico-chimiques des formulations de l'exemple 1

- pH (à température ambiante)

| **Formulation** | **pH** |
|---|---|
| A (illustrative) | 7,0 |
| B (illustrative) | 7,2 |
| C | 7,1 |
| D | 7,0 |
| E | 7,1 |
| F | 7,1 |

- osmolarité

| **Formulation** | **Osmolarité (mOsm/kg)** |
|---|---|
| A (illustrative) | 335 |
| B (illustrative) | 322 |
| C | 324 |
| D | 315 |
| E | 326 |
| F | 335 |

Les formulations A, B, C, D, E et F sont isotoniques et possèdent un pH neutre.

### Exemple 3 : Propriétés rhéologiques des formulations de l'exemple 1

Les propriétés viscoélastiques des formulations A, B, C, D et E sont caractérisées en mesurant l'évolution du module visqueux (G") et du module élastique (G') en fonction de la fréquence (voir figures 1 à 5).

Pour ces 5 formulations, on constate que la fréquence de croisement du module G' et du module G" est proche de celle du liquide synovial sain.

Le tableau ci-dessous donne les valeurs de fréquence de croisement f_{c} pour chaque formulation et pour le liquide synovial sain.

| **Formulation** | **Fréquence de croisement f_{c} (HZ)** |
|---|---|
| A (illustrative) | 0,50 |
| B (illustrative) | 0,32 |
| C | 0,32 |
| D | 0,32 |
| E | 0,33 |
| Liquide synovial sain *(publication de Mazzucco D. et al.)* | 0,41 ± 0,12 |

Comme décrit dans la présente invention :
- Au-dessous de f_{c} : G" > G', le gel est à dominante visqueuse avec une lubrification efficace de l'articulation au repos,
- Au-dessus de f_{c} : G' > G", le gel est à dominante élastique avec une absorption efficace des chocs lorsque le patient court ou saute

### Exemple 4 : Résistance aux dégradations des formulations de l'exemple 1

Pour montrer que la présence d'un polyol dans un gel à base de NaHA permet de réduire la dégradation du gel par une action radicalaire, thermique et mécanique, on a comparé la résistance à la dégradation de gels à base de NaHA avec polyol (formulations de l'exemple 1) et la résistance à la dégradation de gels à base de NaHA sans polyol (= gels références).

Pour les formulations B, C, D et E de l'exemple 1, le gel référence à base de NaHA sans polyol est un gel à base de NaHA non réticulé à 20 mg/ml de NaHA (MM= 2,5x 10⁶ Da, dans du tampon phosphate) - formulation G.

Pour la formulation F de l'exemple 1, le gel référence à base de NaHA sans polyol est un gel à base de NaHA réticulé à 18 mg/ml de NaHA (MM= 2,5x10⁶ Da avant réticulation, dans du tampon phosphate) possédant un taux de réticulation de 6% - formulation H.

Le test de dégradation est effectué en ajoutant un oxydant dans le gel à tester, en homogénéisant à la spatule pendant 1 minute, en se plaçant à la température de *37°C* et en imposant une déformation de 0.3%. La valeur du paramètre Tanδ = G"/G' à 0,7Hz (paramètre caractéristique des propriétés viscoélastiques du gel) est mesurée au cours du temps.

On constate que ce paramètre augmente au cours du temps, synonyme d'une déstructuration progressive du gel. Les valeurs mesurées à t=0 et t=15 min pour les formulations B, C, D, E, F, G et H sont données dans le tableau ci-dessous.

| **Formulation** | **Tanδ (t = 0 min)** | **Tanδ (t = 15 min)** | **Δ Tanδ (%)** |
|---|---|---|---|
| B (illustrative) | 1,10 | 3,34 | +204 % |
| C | 1,08 | 3,13 | +205 % |
| D | 1,19 | 4,63 | +289% |
| E | 1,08 | 2,57 | + 138% |
| F | 0,74 | 0,80 | +8% |
| G | 1,41 | 6,56 | +365 % |
| H (référence) | 0,74 | 0,93 | +26% |

Comme décrit dans la présente invention, chaque formulation B, C, D et E possède une résistance aux dégradations significativement plus importante que celle du gel sans polyol (formulations G). De même, la formulation F possède une résistance aux dégradations significativement plus importante que celle du gel correspondant sans polyol (formulation H).

Par conséquent, les polyols protègent le gel de façon efficace contre les dégradations.

### Exemple 5 : Etude de vieillissement accéléré d'une formulation avec et sans polyol

Deux formulations sont mises en vieillissement accéléré à l'étuve à 40°C :
- formulation B (illustrative) de l'exemple 1: solution à base d'acide hyaluronique et de glycérol
- formulation G sans adjonction d'alcool (décrite dans l'exemple 4)
   - 20 mg d'acide hyaluronique à 2,5 x 10⁶ Da
   - Qsp 1 ml de tampon phosphate

Une mesure de la viscosité zéro (viscosité à cisaillement nul) et une détermination de la fréquence f_{c} de croisement entre le module élastique G' et le module visqueux G" est effectuée à 3 temps (t = 0, 7 jours, 26 jours)

Les résultats obtenus sont donnés dans le tableau ci-dessous :

| **Nombre de jours de vieillissement à 40°C** | **Formulation** | **Viscosité zéro** | **Variation de viscosité zéro par rapport à to (%)** | **f_{c}(Hz)** | **Variation de f_{c} par rapport à to (%)** |
|---|---|---|---|---|---|
| 0 jour | B | 262 | / | 0,32 | / |
| | G | 192 | / | 0,39 | / |
| 7 jours | B | *ND | / | 0,32 | 0% |
| | G | *ND | / | 0,39 | 0% |
| 26 jours | B | 210 | -17 % | 0,37 | +16% |
| | G | 143 | -26 % | 0,50 | +28 % |

| | | | | | |
|---|---|---|---|---|---|
| *ND : non déterminé | | | | | |

On constate que lors du vieillissement accéléré, la perte de viscosité zéro et le décalage de la fréquence de croisement f_{c} sont moins importants dans le cas de la formulation B (formulation avec polyol) que dans le cas de la formulation sans polyol (formulation G).

### Exemple 6 : Comparaison de la rhéologie de 4 produits commerciaux de viscosupplémentation et d'une formulation obtenue selon la présente invention

Les produits testés sont les suivants :

| **Produit** | **Nom commercial** | **Fabricant** | **Concentration en acide hyaluronique (mg/ml)** | **Masse moléculaire de l'acide hyaluronique (Da)** | **Méthode de stérilisation** |
|---|---|---|---|---|---|
| P1 | Synocrom® | CROMA PHARMA | 10 | 2,2 - 2,7.10⁶ | Chaleur humide |
| P2 | Structovial® | CROMA PHARMA | 10 | 2,2 - 2,7.10⁶ | Chaleur humide |

| | | | | | |
|---|---|---|---|---|---|
| P3 | Fermathron® | HYALTECH | 10 | 1.10⁶ | Filtration |
| P4 | Lubravisc® | BOHUS BIOTECH | 10 | 4.10⁶ | Chaleur humide |
| Formulation D de l'exemple 1 | Non applicable | Non applicable | 20 (+ 15 mg/ml de mannitol) | 2,5.10⁶ | Chaleur humide |

Pour les 5 gels testés, les figures 6 - 9 donnent les modules visqueux (G") et élastique (G') en fonction de la fréquence.

On constate que seul le gel selon l'exemple 1 présente une fréquence de croisement (0,50 Hz) voisine de celle du liquide synovial sain (0,41 Hz).

Le tableau ci-dessous regroupe les valeurs des fréquences de croisement f_{c} pour les produits P1 à P4 et pour la formulation D de l'exemple 1.

| **Produit** | **f_{c} (Hz)** |
|---|---|
| Formulation D de l'exemple 1 | 0,32 |
| P1 | 5,8 |
| P2 | 5,0 |
| P3 | 6,3 |
| P4 | 0,09 |

On sait d'après la publication de Mazzucco D. et al. (Citée plus haut) que la fréquence de croisement du liquide synovial sain (0,41 Hz) est en dessous des fréquences observées dans le genou lors de la marche (0,7 Hz) et de la course (3 Hz).

Pour les produits P1 à P3, la fréquence de croisement est bien supérieure à 3 Hz et par conséquent, les produits ne présentent pas une forte élasticité permettant l'absorption des chocs lorsque le genou est en mouvement.

Le produit P4 a une très faible fréquence de croisement, le module élastique est supérieur au module visqueux sur toute la gamme de fréquence 0,1 - 10 Hz. Par conséquent, l'élasticité est importante lorsque le genou est en mouvement mais la lubrification de l'articulation est peu efficace lorsque le patient est au repos.

### Exemple 7 : Comparaison de la résistance aux dégradations de 3 produits commerciaux de viscosupplémentation et d'une formulation obtenue selon la présente invention

Les produits testés sont les suivants :

| **Produit** | **Nom Commercial** | **Fabricant** | **Concentration en acide hyaluronique (mg/ml)** |
|---|---|---|---|
| T1 | Arthrum® | LCA Pharmaceutical | 20 |
| T2 | Ostenil® | TRB Chemedica | 10 |
| T3 | Synocrom® | CROMA PHARMA | 10 |
| Formulation E de l'exemple 1 | Non applicable | Non applicable | 20 (+ 40 mg/ml de sorbitol) |

Le test de dégradation est effectué selon la méthode décrite dans l'exemple 4. La valeur du paramètre G' à 0,7Hz est suivi au cours du temps.

Les courbes de rhéologie ainsi obtenues sont données en figure 10.

On constate que le gel selon la présente invention se dégrade significativement moins rapidement que les 3 produits commerciaux testés.

### Exemple 8 : Mise en évidence de la forte affinité acide hyaluronique/polyol

Afin de démontrer la forte affinité entre l'acide hyaluronique et le polyol et donc la protection du gel par le polyol sur le long terme, une étude de suivi du relarguage d'un polyol par dialyse a été effectuée.

5g de la formulation E (gel à base de 20 mg/ml de NaHA non réticulé et de 40 mg/ml de sorbitol - exemple 1) a été introduit dans une membrane (n°1) de dialyse (Spectra/Pore®, MWCO : 12-14,000).

5g d'une solution tampon phosphate contenant 40 mg/ml de sorbitol a été introduit dans une 2éme membrane (n°2) de dialyse (Spectra/Pore®, MWCO : 12-14,000) - même dimension que la membrane n°1.

Ces membranes ont été disposées dans des flacons respectifs contenant 50 g d'eau purifiée (= bain de dialyse) sous agitation magnétique. Des mesures de concentration en sorbitol par HPLC ont été effectuées dans les bains de dialyse à différents temps afin de suivre la cinétique de relarguage du sorbitol hors de la membrane avec gel ou solution tampon.

Les courbes de suivi de la concentration en sorbitol au cours du temps sont données en figure 11.

La cinétique de relarguage du sorbitol dans un gel est significativement plus lente que dans une solution tampon.

Cette étude met en avant la synergie entre l'acide hyaluronique et le polyol présent dans le gel : la forte affinité acide hyaluronique/polyol permet au polyol d'être présent au sein du gel sur une longue période et la capacité de protection du polyol vis-à-vis du gel permet d'avoir une forte résistance sur le long terme du gel contre les dégradations.

## Revendications

1. Formulation aqueuse injectable stérile, pour une injection intra-articulaire dans le traitement des dégénérescences articulaires, ladite formulation se présentant sous forme de gel contenant de l'acide hyaluronique ou un de ses sels à une teneur de 1-100 mg/ml et éventuellement un ou plusieurs autres polysaccharides d'origine naturelle, et un ou plusieurs polyols à une teneur de 15-100 mg/ml, le ou les polyols étant choisis parmi le propylène glycol, le sorbitol, le mannitol ou un mélange de ceux-ci, le gel stérilisé à l'autoclave ayant une fréquence de croisement du module élastique G' et de module visqueux G" comprise entre 0 et 10 Hz, de préférence 0,41 ± 0,41 Hz, G' étant supérieur à G" à haute fréquence.

2. Formulation selon la revendication 1, dans laquelle l'acide hyaluronique est non réticulé ou sensiblement non réticulé.

3. Formulation selon la revendication 1, dans laquelle l'acide hyaluronique est réticulé.

4. Formulation selon l'une ou l'autre des revendications 1 ou 2, dans laquelle la concentration en acide hyaluronique ou en un de ses sels est de 10 à 25 mg/ml et la concentration en polyol(s) est de 15 à 45 mg/ml.

5. Formulation selon l'une quelconque des revendications 1 à 4 dans laquelle l'acide hyaluronique (ou l'un des sels) seul ou en mélange a une masse moléculaire de 0,1 à 10x10⁶ Da.

6. Formulation selon l'une quelconque des revendications 1 à 5, dans laquelle l'acide hyaluronique a une masse moléculaire de 2 à 3x10⁶ Da.

7. Formulation selon l'une quelconque des revendications 1 à 6 dans laquelle la concentration en acide hyaluronique est de 20 mg/ml et celle en sorbitol est de 40 mg/ml.

8. Formulation selon l'une quelconque des revendications 1 à 2 et 4 à 6 dans laquelle la concentration en hyaluronate de sodium à 2,5 10⁶ Da non réticulé, est de 20 mg/ml et celle en mannitol est de 15 mg/ml, dans une quantité de tampon phosphate suffisante pour 1 ml.

9. Formulation selon l'une quelconque des revendications 1 à 2 et 4 à 6 dans laquelle la concentration en hyaluronate de sodium à 2,5 10⁶ Da non réticulé, est de 20 mg/ml et celle en propylène glycol est de 15 mg/ml, dans une quantité de tampon phosphate suffisante pour 1 ml.

10. Procédé de préparation d'une formulation aqueuse injectable stérile sous forme de gel selon l'une quelconque des revendications 1 à 9 comprenant les étapes consistant à préparer une composition aqueuse contenant l'acide hyaluronique ou un de ses sels, éventuellement un ou plusieurs autres polysaccharides d'origine naturelle, et un ou plusieurs polyols choisis parmi le propylène glycol, le sorbitol; le mannitol ou un mélange de ceux-ci, et à stériliser à l'autoclave la composition ainsi obtenue.

11. Formulation selon l'une quelconque des revendications 1 à 9 pour une utilisation par injection intra-articulaire à raison de 0,1 à 20 ml.

## Patentansprüche

1. Injizierbare sterile wässrige Formulierung für eine intraartikuläre Injektion im Rahmen der Behandlung des Gelenkverschleisses, wobei die Formulierung in Form eines Hyaluronsäure oder ein Salz davon in einer Menge von 1 -100 mg/ml und ggf. mindestens ein Polysaccharid natürlicher Herkunft, und mindestens ein Polyol in einer Menge von 15 - 100 mg/ml enthaltenden Gels vorliegt, wobei das mindestens eine Polyol gewählt ist aus Propylenglykol, Sorbitol, Mannitol oder einer Mischung davon, wobei das mittels eines Autoklavs sterilisierte Gel eine Kreuzfrequenz zwischen Elastizitäts- G' und Viskositätsmodul G" zwischen 0 und 10 Hz, vorzugsweise 0,41 ± 0,41 Hz, aufweist, wobei G' bei hoher Frequenz größer ist als G".

2. Formulierung nach Anspruch 1, wobei die Hyaluronsäure unvernetzt oder im Wesentlichen unvernetzt ist.

3. Formulierung nach Anspruch 1, wobei die Hyaluronsäure vernetzt ist.

4. Formulierung nach einem der Ansprüche 1 oder 2, wobei die Konzentration an Hyaluronsäure oder einem Salz davon 10 - 25 mg/ml und die Polyolkonzentration 15 - 45 mg/ml betragen.

5. Formulierung nach einem der Ansprüche 1 - 4, wobei die Hyaluronsäure (oder Salz davon) allein oder in Mischung eine Molekülmasse von 0,1 - 10 x 10⁶ Da aufweist.

6. Formulierung nach einem der Ansprüche 1 - 5, wobei die Hyaluronsäure eine Molekülmasse von 2 - 3 x10⁶ Da aufweist.

7. Formulierung nach einem der Ansprüche 1 - 6, wobei die Hyaluronsäurekonzentration 20 mg/ml und die Sorbitolkonzentration 40 mg/ml betragen.

8. Formulierung nach einem der Ansprüche 1 - 2 und 4 - 6, wobei, in einer für 1 ml ausreichenden Menge an Phosphatpuffer, die Konzentration an unvernetztem Natriumhyaluronat bei 2,5 x 10⁶ Da 20 mg/ml und die Mannitolkonzentration 15 mg/ml betragen.

9. Formulierung nach einem der Ansprüche 1 - 2 und 4 - 6, wobei, in einer für 1 ml ausreichenden Menge an Phosphatpuffer, die Konzentration an unvernetztem Natriumhyaluronat bei 2,5 x 10⁶ Da 20 mg/ml und die Propylenglykolkonzentration 15 mg/ml betragen.

10. Verfahren zur Präparation einer injizierbaren sterilen wässrigen Formulierung in Gelform nach einem der Ansprüche 1 - 9, umfassend die Schritte: Präparieren einer Hyaluronsäure oder ein Salz davon, ggf. mindestens anderes Polysaccharid natürlicher Herkunft, und mindestens ein aus Propylenglykol, Sorbitol, Mannitol oder einer Mischung davon gewähltes Polyol enthaltenden wässrigen Zusammensetzung, und Sterilisieren der resultierenden Zusammensetzung mittels eines Autoklavs.

11. Formulierung nach einem der Ansprüche 1 - 9 zur Verwendung in einer intraartikulären Injektion in einer Menge von 0,1 - 20 ml.

## Claims

1. Sterile injectable aqueous formulation for intraarticular injection in the treatment of joint degeneration, wherein the formulation is in the form of a gel containing hyaluronic acid or a salt thereof in an amount of 1 - 100 mg/ml and optionally one or more other polysaccharides of natural origin, and one or more polyols in an amount of 15 -100 mg/ml, wherein the polyol(s) are selected from propylene glycol, sorbitol, mannitol, or a mixture thereof, wherein the autoclave-sterilised gel has a crossover frequency between elastic modulus G' and viscous modulus G" between 0 and 10 Hz, preferably 0.41 ± 0.41 Hz, wherein G' is greater than G " at high frequency.

2. Formulation according to claim 1, wherein the hyaluronic acid is not crosslinked or substantially not crosslinked.

3. Formulation according to claim 1, wherein the hyaluronic acid is crosslinked.

4. Formulation according to either of claims 1 or 2, wherein the concentration of hyaluronic acid or one of its salts is 10 - 25 mg/ml, and the polyol concentration is 15 - 45 mg/ml.

5. Formulation according to any of claims 1 - 4, wherein the hyaluronic acid (or one of its salts), alone or in mixture, has a molecular mass of 0.1 - 10 x 10⁶ Da.

6. Formulation according to any of claims 1 - 5, wherein the hyaluronic acid has a molecular mass of 2 - 3 x 10⁶ Da.

7. Formulation according to any of claims 1 - 6, wherein the hyaluronic acid concentration is 20 mg/ml and the sorbitol concentration is 40 mg/ml.

8. Formulation according to any of claims 1 - 2 and 4 - 6, wherein the non-crosslinked 2.5 x 10⁶ Da sodium hyaluronate concentration is 20 mg/ml, and the mannitol concentration is 15 mg/ml in an amount of phosphate buffer sufficient for 1 ml.

9. Formulation according to any of claims 1 - 2 and 4 - 6, wherein the non-crosslinked 2.5 x 10⁶ Da sodium hyaluronate concentration is 20 mg/ml, and the propylene glycol concentration is 15 mg/ml in an amount of phosphate buffer sufficient for 1 ml.

10. Method for preparing a sterile injectable aqueous formulation in gel form according to any of claims 1 - 9, comprising the following steps: preparing an aqueous composition containing hyaluronic acid or one of its salts, and optionally one or more other polysaccharides of natural origin, and one or several polyols selected from propylene glycol, sorbitol, mannitol, or a mixture thereof, and sterilising the composition thus obtained by means of an autoclave.

11. Formulation according to any of claims 1 - 9 for use in an intraarticular injection at a ratio of 0.1 -20 ml.
